# EUROPÄISCHE PATENTANMELDUNG

(11) **EP 0 916 948 A2**
(43) Veröffentlichungstag der Anmeldung: **19.05.1999**
(21) Anmeldenummer: 98121793.8
(22) Anmeldetag: 16.11.1998
(51) Int. Cl.: G01N 33/48

(54) **Verbessertes Verfahren zum Nachweis von PSA-ACT-Komplexen**

(30) Priorität: 17.11.1997 DE 19750916
(71) Anmelder: Roche Diagnostics GmbH, 68305 Mannheim (DE)
(72) Erfinder: Kientsch-Engel, Rosemarie Dr., 82340 Feldafing (DE); Mössner, Ellen Dr., 82327 Tutzing (DE); Nilsson, Olle Dr., 41763 Göteborg (SE)
(74) Vertreter: Böhm, Brigitte, Dipl.-Chem. Dr.

(57) **Zusammenfassung**

Zur immunologschen Bestimmung von PSA-ACT-Komplexen in einer zu untersuchenden Probe, bei welchem die Probe in Kontakt gebracht wird mit mindestens einem die Komplexe oder deren Bestandteile spezifisch bindenden Antikörper oder dessen spezifisch bindenden Fragmenten, wobei der Antikörper an eine feste Phase gebunden oder bindbar ist, wird erfindungsgemäß mindestens während des Kontakts zwischen der Probe und der festen Phase in einer Testflüssigkeit gearbeitet, die 0,5 bis 2,5 mol/l Alkalichlorid enthält. Ein erfindungsgemäßes Puffersystem für einen PSA-ACT-Test enthält neben anderen üblichen Puffersubstanzen 0,5 bis 2,5 mol/l, vorzugsweise 1,5 bis 2,0 mol/l Alkalichlorid. Ein erfindungsgemäßer Testkit für den Nachweis von PSA-ACT-Komplexen im Serum von Patienten enthält
a) einen für PSA-spezifischen Antikörper und
b) einen für ACT-spezifischen Antikörper, wobei einer der Antikörper a) und b) an eine feste Phase bindbar oder gebunden ist, und der andere Antikörper a) oder b) eine Markierung aufweist, über die die Nachweisreaktion erfolgt, und als Puffer mindestens ein Puffersystem nach einem der Ansprüche 10 bis 15.

## Beschreibung

Die vorliegende Erfindung betrifft ein Verfahren zur immunologischen Bestimmung von PSA-ACT-Komplexen, Puffersysteme für derartige Tests und einen Testkit für den Nachweis von PSA-ACT-Komplexen im Serum von Patienten.

Das Prostata-spezifische Antigen, PSA, wird von Prostata-Epithelzellen gebildet und hat die enzymatische Aktivität einer neutralen Serinprotease. PSA ist Bestandteil der Samenflüssigkeit, geringe Mengen gehen aber auch in den menschlichen Blutkreislauf über. Das enzymatisch aktive PSA wird im Serum durch verschiedene Serinproteaseinhibitoren durch Bildung von kovalenten Komplexen inaktiviert. Die Hauptmenge des immunologisch nachweisbaren PSA ist im Serum an α1-Antichymotrypsin (zu 60 bis 95 %) gebunden.

α1-Antichymotrypsin (ACT) ist ein Glykoprotein, welches eine wichtige Rolle bei der Kontrolle von Entzündungen spielt. ACT kommt im humanen Serum in 10.000-fach höherer Konzentration vor als PSA.

Das komplexierte PSA-ACT liegt neben freiem PSA als Hauptform des immunologisch nachweisbaren Gesamt-PSA im Serum vor. Es wurde festgestellt, daß im Vergleich zu Patienten mit benigner Prostata-Hyperplasie erhöhte Werte an komplexiertem PSA vorwiegend bei Patienten mit malignem Prostatatumor auftreten (japanische Offenlegungsschrift 62-46263).

Der Nachweis von PSA-ACT dient daher u.a. der Auffindung und dem Nachweis von malignen Prostata-Karzinomen sowie einer Überwachung von therapeutischen Maßnahmen. Die Menge des vorhandenen PSA-ACT korreliert hierbei ab einem Grenzwert sehr stark mit dem Vorhandensein bzw. der Malignizität und Größe von Prostata-Karzinomen.

PSA-ACT-Komplexe werden daher bereits mittels Sandwich-Assays unter Verwendung von spezifischen Antikörpern nachgewiesen. Hierbei wird zum einen ein Festphasen-gekoppelter Antikörper verwendet, der spezifisch ist für eine der beiden Komplexkomponenten, sowie ein markierter Antikörper, der für die andere der beiden Komplexkomponenten spezifisch ist, wobei der Nachweis letztendlich über die Markierung des zweiten Antikörpers erfolgt (z.B. WO92/01936, JP62-46263, Pettersson et al., Clin. Chem. 41/10 (1995), 1480-1488, Leinonen et al., Clin. Chem. 39/10 (1993), 2098-2103).

Leider hat sich nun herausgestellt, daß im Serum Substanzen enthalten sind, die diesen Nachweis empfindlich stören können. Es handelt sich hierbei vorwiegend um Kathepsin-G, welches ebenso wie PSA eine Serinprotease ist und auch von dem Inhibitor α1-Antichymotrypsin (ACT) gebunden und dadurch inaktiviert wird. Kathepsin-G hat aber zusätzlich die unangenehme Eigenschaft, sehr klebrig zu sein und sich beispielsweise bei immunologischen Tests an die Festphase zu binden. In Gegenwart von Kathepsin G, welches insbesondere bei Vorliegen von Entzündungen in großen Mengen gebildet wird, kommt es daher bei den gängigen Tests zur Ausbildung von Komplexen zwischen der Festphase, Kathepsin-G-ACT und dem markierten anti-ACT-Antikörper, wodurch das Vorhandensein von PSA-ACT-Komplexen vorgetäuscht und fälschlich erhöhte Werte gemessen werden (siehe z. B. Leionen, 1993, supra, wo über Tests berichtet wird, bei denen PSA-ACT/PSA_{gesamt} mit größer 1 gemessen wurde und sogar bei Seren von Frauen falsch positive Werte aufgefunden wurden).

Aufgabe der vorliegenden Erfindung war es daher, die Spezifität des Nachweises von PSA-ACT-Komplexen zu erhöhen und Störungen insbesondere durch Kathepsin-G zu vermeiden.

Gelöst wird diese Aufgabe durch ein Verfahren zur immunologischen Bestimmung von PSA-ACT-Komplexen in einer zu untersuchenden Probe, bei welchem die Probe in Kontakt gebracht wird mit einem die Komplexe oder deren Bestandteile spezifisch bindenden Antikörper oder dessen spezifisch bindenden Fragmenten, wobei der Antikörper an eine feste Phase gebunden oder bindbar ist, dadurch gekennzeichnet, dass zumindest, während Kontakt zwischen der Probe und der festen Phase besteht, in einer Testflüssigkeit gearbeitet wird, die 0,5 bis 2,5 mol/l eines Alkalichlorids enthält.

Die auf das Vorhandensein von PSA-ACT-Komplexen zu untersuchende Probe ist eine Körperflüssigkeit, in der sich bei Vorliegen maligner Prostatatumore erhöhte PSA-ACT-Komplex-Konzentrationen finden.

Im Rahmen der Erfindung wurde festgestellt, daß überraschenderweise ein relativ hoher Salzgehalt im verwendeten Testpuffer unspezifische Bindungen weitestgehend verhindert und bei den eingesetzten Antikörpern die immunologische Reaktion nicht stört. Mit Hilfe des erfindungsgemäßen Verfahrens werden Werte erhalten, welche eine sehr hohe Korrelation des berechneten Wertes aus der Differenz des totalen PSA und freien PSA zu dem tatsächlich gemessenen Wert ergeben, was für die Qualität des neuen Verfahrens spricht.

Im Rahmen der vorliegenden Erfindung verwendet man vorzugsweise einen Puffer, der 1,5 bis 2,0 mol/l Alkalichlorid enthält. Besonders bevorzugt ist es hierbei, als Alkalichloride Natrium- oder Kaliumchlorid einzusetzen.

Desweiteren wurde festgestellt, daß ein Zusatz eines Alkalitartrats zum Versuchspuffer darüber hinaus von Vorteil ist. Erfindungsgemäß wird vorteilhaft 0,01 bis 0,05 mol/l Alkalitartrat verwendet. Ein besonders bevorzugtes Tartrat ist das Natriumtartrat. Dieses wird insbesondere in Mengen von 0,02 bis 0,03 mol/l im Puffer eingesetzt.

Eine weitere Möglichkeit, falsch-positive Resultate weiter zu verringern, liegt darin, daß man einen Puffer verwendet, der zusätzlich noch modifiziertes Thermo-RSA (Thermo-Rinderserumalbumin) (z.B. WO95/17668) enthält und zwar vorzugsweise in einer Menge von 0,2 bis 0,5 g/l.

Für Immunoassays geeignete Festphasen sind dem Fachmann bekannt. Lediglich beispielhaft sollen hier die gängigsten Festphasen genannt werden, wie Kunststoffröhrchen (Kunststofftubes), Polystyrolkügelchen, Latexpartikel, Mikrotiterplatten, Glasbeads etc.

Möglichkeiten zur Bindung von Antikörpern an derartige Festphasen sind dem Fachmann ebenfalls bekannt. Beispielhaft sei hier auf die Bindungspaare Biotin/(Strept-)Avidin, Hapten/Antikörper, Lectin/Kohlehydrat verwiesen, von denen jeweils ein Partner an die Festphase gekoppelt wird und der andere Partner mit dem Antikörper assoziiert vorliegt.

Diese Bindungspaare sind aber nur als Beispiele zu verstehen, andere Bindungspaare sind ebenso einsetzbar im Rahmen der Erfindung, wie eine direkte, z. B. adsorptive Bindung des Fangantikörpers an die Festphase.

Nach der Kopplung der Komplexe über den Antikörper an die Festphase kann die Detektion der gebundenen Komplexe in beliebiger, dem Fachmann bekannter Weise erfolgen. In einer bevorzugten Ausführungsform wird ein Immunoassay angewandt, bei dem die Schritte
a) Kopplung eines für (1) PSA oder (2) ACT spezifischen Antikörpers an eine feste Phase,
b) Zugabe einer zu untersuchenden Probe zu der festen Phase und
c) Nachweis des Vorhandenseins von PSA-ACT-Komplexen durch Zugabe eines markierten Antikörpers, der im Fall a) (1) für ACT und im Fall a) (2) für PSA spezifisch ist und Detektion über die Markierung dieses Antikörpers,
in geeigneter Reihenfolge durchgeführt werden, wobei man erfindungsgemäß zumindest in Schritt b) einen Puffer einsetzt, mit Hilfe dessen die Testflüssigkeit auf einen Gehalt an 0,5 bis 2,5 mol/l eines Alkalichlorids eingestellt wird.

Das bevorzugte erfindungsgemäße Verfahren beinhaltet also einen Sandwich-Immunoassay, wie er im Prinzip auch bisher bereits angewandt wird. Alle für Sandwich-Immunoassays geeigneten Testformate einschließlich bekannter Festphasen, Systeme zur Kopplung von Fangantikörpern an Festphasen, Nachweise mittels Markierungen des Nachweisantikörpers sind im Rahmen der Erfindung einsetzbar mit der Maßgabe, dass zumindest bei der Kontaktierung der zu untersuchenden Probe mit der Festphase, dem festphasengebundenen Antikörper bzw. mit dem Antikörper, der an die Festphase zu binden ist, der angegebene Salzgehalt vorliegt. Auch die Reihenfolge durch Durchführung der Schritte a) und b) ist im bevorzugten erfindungsgemäßen Verfahren nicht kritisch.

Auch die Auswahl der mit dem Nachweisantikörper im Schritt (c) assoziierten Markierung ist im Rahmen der Erfindung nicht kritisch. Alle Markierungen, die generell für Sandwich-Immunoassays geeignet sind, können auch im vorliegenden Verfahren eingesetzt werden. Dabei können direkte oder indirekte Markierungen zum Einsatz kommen, wobei unter indirekten Markierungen solche zu verstehen sind, die wiederum über eine weitere Substanz nachgewiesen werden, wie ein Hapten, das über einen weiteren Antikörper nachgewiesen wird (z.B. DIG-Anti-DIG).

Beispielhaft für direkte Markierungen seien Enzymmarkierung, zur Elektrolumineszenz fähige Label, wie Ru-Chelate, Goldmarkierung und Fluorophore genannt.

In einer weiteren bevorzugten Ausführungsform der Erfindung führt man die Schritte a) und b) des erfindungsgemäßen Verfahrens gleichzeitig durch und zwar in einem Puffer, der die benötigten Komponenten Salz und gegebenenfalls Tartrat oder/und Thermo-RSA enthält. Diese Vorgehensweise erleichtert und beschleunigt den erfindungsgemäßen Nachweis.

Im Rahmen der vorliegenden Erfindung liegt es auf der Hand, dass Antikörper verwendet werden, welche durch die Salzkonzentration des Puffers nicht in ihrer Spezifität und Bindefähigkeit beeinträchtigt werden. Es ist durch einfache Versuche leicht festzustellen, ob ein in Erwägung gezogener Antikörper dieser Bedingung entspricht. Es ist desweiteren von Vorteil, monoklonale Antikörper im erfindungsgemäßen Verfahren einzusetzen. Schließlich können im Rahmen der vorliegenden Erfindung auch Antikörper eingesetzt werden, die PSA bzw. ACT in der komplexierten PSA/ACT-Form spezifisch binden. Derartige Antikörper sind von der erfindungsgemäßen Angabe "für PSA- bzw. -ACT spezifischer Antikörper" mitumfasst und z.B. in der deutschen Patentanmeldung 196 41 560.8 beschrieben, auf die hiermit Bezug genommen wird. Es ist für den Fachmann selbstverständlich, dass auch für diesen Fall natürlich nur dann ein Sandwich-Assay erfolgen kann, wenn beide verwendete Antikörper unterschiedliche Epitope des PSA/ACT-Komplexes erkennen oder nur ein Komplex-bindender Antikörper und ein für einen der beiden Komplexteile spezifischer Antikörper verwendet wird. Bei allen oben beschriebenen Arten von Antikörpern können darüber hinaus auch die für Immunoassays geeigneten Fragmente (Fab, Fab', F(ab)₂') oder aber auch chimäre Antikörper zum Einsatz kommen.

Alle das Testformat betreffenden Angaben sind lediglich zur Erläuterung vorhanden und nicht als abschließende Beschreibung aufzufassen. Im Rahmen der vorliegenden Erfindung sind, wie oben ausgeführt, alle Immunoassays und insbesondere Sandwich-Assays geeignet, solange mit der erfindungsgemäßen Konzentration an Alkalichlorid (zumindest in Schritt b)), gegebenenfalls aber auch bei weiteren Schritten des Assays gearbeitet wird.

Im Rahmen der Erfindung ist es aber ebenso möglich, einen Test durchzuführen, indem z.B. ein nur für den Komplex PSA-ACT spezifischer Antikörper als Fangantikörper eingesetzt wird und nach Art eines kompetitiven Assays martkierte PSA-ACT-Komplexe zugesetzt werden. Alle weiteren geeigneten Testformate, bei denen durch unspezifische Bindung von in der Probe enthaltenen Substanzen an die feste Phase falsch positive Ergebnisse bezüglich PSA-ACT-Komplexen erhalten werden, sind aber ebenfalls positiv durch die erfindungsgemäße Zugabe von Salz(en) oder/und anderen Substanzen zur Testflüssigkeit zu beeinflussen und daher im Rahmen der vorliegenden Erfindung geeignete Verfahren.

Ein weiterer Gegenstand der vorliegenden Erfindung ist ein Puffersystem für einen PSA-ACT-Test, enthaltend neben anderen üblichen Puffersubstanzen 0,5 bis 2,5 mol/l und insbesondere 1,5 bis 2,0 mol/l Alkalichlorid. Im erfindungsgemäßen Puffersystem ist es bevorzugt, dass es als Alkalichlorid Natrium- oder/und Kaliumchlorid enthält, welche sich als besonders geeignet erwiesen haben. Zusätzlich enthält das erfindungsgemäße Puffersystem bevorzugt ein Alkalitartrat und zwar insbesondere in Mengen von 0,01 bis 0,05 mol/l und vorzugsweise 0,02 bis 0,03 mol/l. Das erfindungsgemäß besonders bevorzugte Alkalitartrat ist Natriumtartrat. Das erfindungsgemäße Puffersystem enthält desweiteren vorteilhaft modifiziertes Thermo-RSA, insbesondere in Mengen von 0,2 bis 0,5 g/l.

Mit Hilfe des erfindungsgemäßen Puffersystems können PSA-ACT-Tests, welche bevorzugt als Sandwich-Immunoassay ausgestaltet sind, aber nicht darauf beschränkt sind, praktisch ohne Störungen durch andere im Blut, Serum oder anderen Körperflüssigkeiten enthaltene Substanzen durchgeführt werden. Der Salzgehalt des Puffersystems sowie die Anwesenheit von modifiziertem Thermo-RSA verhindert, daß unspezifisch bindende Substanzen, wie z.B. das "klebrige" Kathepsin-G, unspezifische Überbrückungen der Antikörper bewirken oder unspezifisch eine Bindung des markierten Antikörpers oder einer anderen Nachweissubstanz an die Festphase bewirken.

Ein weiterer Gegenstand der Erfindung ist daher die Verwendung des erfindungsgemäßen Puffersystems zur spezifischen immunologischen Bestimmung von PSA-ACT-Komplexen in einer zu untersuchenden Probe.

Die zu untersuchende Probe ist eine Körperflüssigkeit, welche bei Vorliegen eines malignen Prostatatumors eine erhöhte Konzentration des PSA-ACT-Komplexes aufweist, insbesondere Vollblut, Plasma oder Serum, wobei bevorzugt Serum untersucht wird.

Ein weiterer Gegenstand der vorliegenden Erfindung ist ein Testkit für den Nachweis von PSA-ACT-Komplexen im Serum von Patienten. Der erfindungsgemäß bevorzugte Testkit enthält für den PSA-ACT-Nachweis als Sandwich-Assay geeignete Substanzen, also
a) einen für PSA-spezifischen Antikörper und
b) einen für ACT-spezifischen Antikörper, wobei einer der Antikörper an eine feste Phase bindbar oder gebunden ist, und der andere Antikörper eine Markierung aufweist, über die die Nachweisreaktion erfolgt. Der erfindungsgemäße Testkit enthält darüberhinaus als Puffer ein erfindungsgemäßes Puffersystem, enthaltend Alkalichlorid, insbesondere Natrium- oder Kaliumchlorid, sowie gegebenenfalls Alkalitartrat, vorzugsweise Natriumtartrat, sowie gegebenenfalls modifiziertes Thermo-RSA. Die im Puffersystem enthaltenen Mengen dieser Substanzen sowie die bevorzugt einzusetzenden Mengen sind bereits in den obigen Ausführungen genannt.

Bevorzugt enthält der erfindungsgemäße Testkit auch eine feste Phase, an die einer der beiden Antikörper, der Fangantikörper, gebunden oder an die er bindbar ist, mittels z.B. eines Bindungspaares, wie oben für das erfindungsgemäße Verfahren bereits ausgeführt.

Die Art der festen Phase ist nicht limitierend, alle für Sandwich-Assays geeigneten Festphasen können im Testkit vorhanden sein.

Ebenso gelten für die verwendeten Antikörper sowie die Markierung des einen Antikörpers (Nachweisantikörper) die bereits oben für das erfindungsgemäße Verfahren gemachten Erläuterungen und die gegebenen Beispiele.

Mit Hilfe des erfindungsgemäßen Verfahrens, des Puffersystems sowie des Testkits ist ein sehr aussagekräftiger und störungsfreier immunologischer Nachweis von PSA-ACT-Komplexen möglich. So konnte für den erfindungsgemäßen Sandwich-Test festgestellt werden, dass die Summe des gemessenen PSA-ACT-Komplexes und des freien PSA (nach bekannten Methoden bestimmt) mit dem Gesamt-PSA übereinstimmt. Auch bei Frauen, selbst bei Vorliegen von Entzündungen, wodurch die Menge an Kathepsin G, ACT und des Komplexes ACT-Kathepsin G stark erhöht ist, werden richtigerweise PSA-ACT-Werte von praktisch Null gemessen mit Hilfe des erfindungsgemäßen Verfahrens. Die eingesetzten Salzgehalte stören den Nachweis nicht, so daß die erfindungsgemäßen Puffersysteme sich nur positiv auswirken.

Die folgenden Beispiele sollen die Erfindung weiter erläutern.

### Beispiel 1

### Effekt von Natriumchlorid auf Teststörung durch Kathepsin G

Die Entstörung des PSA-ACT-Nachweises wurde folgendermaßen untersucht:
Als Festphase dienten Streptavidin beschichtete Polystyroltubes (Boehringer Mannheim GmbH, Best.Nr. 1 144 553). Zum Inkubationspuffer des Enzymun-Tests^{R} PSA (Boehringer Mannheim GmbH, Best.Nr. 1 555 332) Flasche 1 ohne Tartrat wurde der anti-PSA monoklonale Antikörper Klon M10 als biotinyliertes Fab-Fragment in der Konzentration von 1,5 µg/ml und steigende Mengen NaCl zugesetzt (= Puffer 1). Zu der Variante mit 2 mol/l NaCl wurden noch zusätzlich 0,025 bis 0,2 mol/l Na-Tartrat zugefügt (= Puffer 2). Die Testdurchführung erfolgte am Analysenautomaten ES 300 (Boehringer Mannheim GmbH) mit den Inkubationszeiten 45, 30 und 30 min für die Proben-, Konjugat- bzw. Substratreaktion in Volumina von jeweils 700 µl; zwischen den einzelnen Inkubationsschritten wurde mit Intensität 8 gewaschen (Enzymun-Test^{R} Waschlösung, Boehringer Mannheim GmbH, Best.Nr. 1 059 475).

Das PSA-ACT Standardmaterial, gekauft von der Fa. Scripps (Kat.Nr. P 0624. CH. 516664), wurde in Konzentrationen von 0, 0,61, 2,44, 13,6 und 54,67 ng/ml eingesetzt. Als monoklonaler Nachweisantikörper diente anti-ACT Klon 53 nach Konjugation des IgG mit Meerrettich-Peroxidase. Die Farbreaktion erfolgte in üblicher Weise mit ABTS^{R} (2, 2'-Azino-di-[3-ethylbenzthiazolin-sulfonsäure)6)]diammoniumsalz) 1, 9 mmol/l und 3, 2 mmol/l Natriumperborat in Phosphat-/Citratpuffer 100 mmol/l, pH 4,4.

Zu gepooltem Serum von gesunden männlichen bzw. weiblichen Blutspendern wurde 0,5 bis 5 mg Kathepsin G/l zugesetzt und der Einfluss der verschiedenen Puffersysteme untersucht. Es zeigte sich (siehe Tabelle 1), dass der Zusatz von 2 mol/l NaCl die Teststörung stark vermindert (Puffer 1) und die weitere Zugabe geringer Mengen von Natrium-Tartrat die Störung weiter verringert (Puffer 2).

**Tabelle 1**

| **Probe** | Puffer 1 + NaCl [mol/l] | | | | | Puffer 2 + Di-Natrium-Tartrat (mol/l] | | |
|---|---|---|---|---|---|---|---|---|
| | 0,15 | 0,5 | 1,0 | 1,5 | 2,0 | 0,025 | 0,1 | 0,2 |
| Männerserum* + Kathepsin G [mg/l) | | | | | | | | |
| 0 | 0,105 | 0,101 | 0,095 | 0,094 | 0,087 | 0,087 | 0,096 | 0,100 |
| 0,5 | 0,114 | 0,106 | 0,101 | 0,099 | 0,086 | 0,087 | 0,098 | 0,102 |
| 1 | 0,130 | 0,114 | 0,113 | 0,105 | 0,091 | 0,089 | 0,102 | 0,110 |
| 2 | 0,152 | 0,122 | 0,119 | 0,106 | 0,095 | 0,091 | 0,110 | 0,113 |
| 5 | 0,196 | 0,145 | 0,139 | 0,118 | 0,108 | 0,094 | 0,126 | 0,126 |

| Frauenserum ** + Kathepsin G [mg/l] | | | | | | | | |
|---|---|---|---|---|---|---|---|---|
| 0 | 0,002 | 0,004 | 0,002 | 0,002 | 0,002 | 0,002 | 0,003 | 0,008 |
| 0,5 | 0,006 | 0,010 | 0,004 | 0,004 | 0,004 | 0,004 | 0,003 | 0,008 |
| 1 | 0,010 | 0,011 | 0,007 | 0,006 | 0,004 | 0,004 | 0,004 | 0,008 |
| 2 | 0,016 | 0,012 | 0,009 | 0,007 | 0,003 | 0,003 | 0,004 | 0,009 |
| 5 | 0,019 | 0,018 | 0,015 | 0,010 | 0,006 | 0,004 | 0,008 | 0,012 |

| | | | | | | | | |
|---|---|---|---|---|---|---|---|---|
| * = gepooltes Serum von gesunden männlichen Blutspendern | | | | | | | | |
| ** = gepooltes Serum von gesunden weiblichen Blutspendern Angaben in Extinktionseinheiten, gemessen bei 422 nm | | | | | | | | |

### Beispiel 2

### Einfluss anderer Alkalisalze auf die Entstörung des PSA-ACT-Tests

Zu dem Inkubationspuffer des Enzymun-Tests^{R} PSA wurden statt NaCl andere Alkalisalze zugesetzt und die Auswirkung auf die PSA-ACT-Bestimmung in Gegenwart steigender Mengen Kathepsin G gemessen. Die übrige Testdurchführung entspricht der in Beispiel 1. Das Ergebnis ist in Tabelle 2 zusammengefasst.

Dieser Versuch zeigt, dass KCl fast genauso wirksam wie NaCl die Teststörung eliminiert. KJ und KSCN waren ungeeignet, da sie den Test an sich störten (siehe Tabelle 2, unterer Teil PSA-Standard). LiCl entstört nicht ausreichend.

**Tabelle 2**

| Probe | PSA-Puffer mit Zusatz von | | | | |
|---|---|---|---|---|---|
| Serum* mit Kathepsin G [mg/l] | - | KCl 1,5 mol/l | KJ 1,5 mol/l | LiCl 0,5 mol/l | KSCN 1 mol/l |
| 0 | 0,016 | 0,011 | 0,011 | 0,010 | 0,020 |
| 0,5 | 0,027 | 0,020 | 0,018 | 0,026 | 0,033 |
| 1,0 | 0,048 | 0,022 | 0,024 | 0,040 | 0,043 |
| 2,0 | 0,081 | 0,035 | 0,037 | 0,077 | 0,063 |
| 5,0 | 0,21 | 0,065 | 0,068 | 0,175 | 0,146 |

| PSA-ACT Standard | | | | | |
|---|---|---|---|---|---|
| 0 ng/ml | 0,005 | 0,012 | 0,007 | 0,005 | 0,010 |
| 54,7 ng/ml | 4,128 | 4,272 | 1,680 | 4,144 | 1,411 |

| | | | | | |
|---|---|---|---|---|---|
| *gepooltes Serum gesunder, männlicher Blutspender Angaben in Extinktionseinheiten, gemessen bei 422 nm | | | | | |

### Beispiel 3

### Zusatz von modifiziertem Thermo-RSA vermindert die Störung im PSA-ACT-Test

Vergleicht man zwei Inkubationspuffer mit unterschiedlicher Thermo-RSA-Konzentration, wie beispielsweise die Inkubationspuffer (Fl. 1) der Enzymun-Tests^{R} NSE (Boehringer Mannheim GmbH, Best.Nr. 1660438) und PSA (Boehringer Mannheim GmbH, Best.Nr. 1555332), die modifiziertes Thermo-RSA (beschrieben in WO95/17668) in Konzentrationen von 0,05 bzw. 0,35 g/l enthalten und in ihrer übrigen Zusammensetzung gleich sind, so wird deutlich (siehe Tabelle 3), dass diese Komponente wesentlich zur Testentstörung beiträgt.

Die übrigen Testbestandteile und die Durchführung entsprechen derjenigen von Beispiel 1.

**Tabelle 3**

| Probe Serum* mit Kathepsin G [mg/l] | Inkubationspuffer mit Thermo-RSA | | |
|---|---|---|---|
| | 0,05 g/l | 0,35 g/l | 0,035 g/l + 2 mol/l NaCl |
| 0 | 0,116 | 0,105 | 0,087 |
| 0,5 | 0,135 | 0,114 | 0,087 |
| 1,0 | 0,185 | 0,130 | 0,089 |
| 2,0 | 0,256 | 0,152 | 0,091 |
| 5,0 | 0,352 | 0,196 | 0,094 |

| | | | |
|---|---|---|---|
| * gepooltes Serum gesunder, männlicher Blutspender Angaben in Extinktionseinheiten, gemessen bei 422 nm | | | |

### Beispiel 4

### Serumspiegel PSA-ACT bei verschiedenen Patienten-/Probandengruppen

Mit der in Beispiel 1 beschriebenen Testversion unter Verwendung des Inkubationspuffers mit Zusatz von 2 mol/l NaCl und 0,025 mol/l Natriumtartrat wurden die PSA-ACT-Spiegel in den Seren verschiedener Probanden- bzw. Patientengruppen bestimmt. Ergebnis siehe Tabelle 4.

**Tabelle 4**

| PSA-ACT-Gehalt von Seren | | | | | |
|---|---|---|---|---|---|
| Gruppe | Kontrollen männlich | Kontrollen weiblich | Benigne Prostatahyper-plasie | Prostatacarcinom | Andere Erkrankungen, männliche Patienten |
| Anzahl Proben PSA-ACT [ng/ml] | 70 | 41 | 51 | 61 | 85 |
| Mittelwert | 1,08 | 0,01 | 2,76 | 8,49 | 1,29 |
| Median | 0,75 | 0,00 | 1,83 | 5,27 | 0,66 |
| Minimalwert | 0,00 | 0,00 | 0,00 | 0,00 | 0,00 |
| Maximalwert | 5,26 | 0,04 | 13,03 | 69,00 | 29,10 |

### Beispiel 5

### Vergleich berechnetes/gemessenes PSA-ACT

Wenn man annimmt, dass sich der PSA gesamt-Gehalt einer Probe im Wesentlichen aus den beiden Komponenten PSA frei und PSA-ACT zusammensetzt, kann als Test für die Plausibilität des PSA-ACT-Ergebnisses die Differenz aus [PSA gesamt minus PSA frei] berechnet und dieses Ergebnis mit dem in denselben Proben bestimmten PSA-ACT verglichen werden.

In den für Beispiel 4 eingesetzten Proben (außer den weiblichen Kontrollen) wurde daher zusätzlich mit den entsprechenden Enzymun-Tests^{R} PSA gesamt und PSA frei bestimmt und die Korrelation der Differenz aus diesen beiden Parametern zu PSA-ACT berechnet. Das Ergebnis ist in Tabelle 5 zusammengefasst.

**Tabelle 5**

| Parameter 1 | Parameter 2 | Kollektiv | Anzahl Werte | r | m | b |
|---|---|---|---|---|---|---|
| PSA-ACT (= Y-Wert] | tPSA-fPSA (= X-Wert) | alle | 264 | 0,994 | 1,010 | 0,283 |
| | | K, männl. | 70 | 0,988 | 1,153 | 0,092 |
| | | BPH | 51 | 0,955 | 1,211 | 0,191 |
| | | PCa | 60 | 0,997 | 0,996 | 0,249 |
| | | And.K. | 83 | 0,998 | 1,024 | 0,187 |
| tPSA = PSA gesamt, fPSA = PSA frei K, männl. = männliche gesunde Kontrollen BPH = Benigne Prostatahyperlasie PCa = Prostatacarcinom And.K. = andere Erkrankungen männlicher Patienten r = Korrelationskoeffizient m = Steigung der Korrelationsgeraden b = Achsenabschnitt der Korrelationsgeraden | | | | | | |

Mit allen Proben war die Korrelation von berechnetem und gemessenem PSA-ACT nahe 1. Dies zeigt ein plausibles Ergebnis für PSA-ACT, das mit der neuen Bestimmungsmethode erzielt wurde.

## Patentansprüche

1. Verfahren zur Durchführung eines heterogenen Immunoassays zur immunologischen Bestimmung von PSA-ACT-Komplexen in einer zu untersuchenden Probe, bei welchem die Probe in Kontakt gebracht wird mit mindestens einem die Komplexe oder deren Bestandteile spezifisch bindenden Antikörper oder dessen spezifisch bindenden Fragmenten, wobei der Antikörper oder seine Fragmente an eine feste Phase gebunden oder bindbar ist,
**dadurch gekennzeichnet,** daß
mindestens während des Kontaktes zwischen der Probe und der fe-sten Phase in einer Testflüssigkeit gearbeitet wird, die 0,5 bis 2,5 mol/l Alkalichlorid enthält.

2. Verfahren nach Anspruch 1,
**dadurch gekennzeichnet,** daß man
dem Puffer zusätzlich ein Alkalitartrat in einer Menge zusetzt, dass im Versuchsansatz 0, 01 bis 0, 05 mol/l Alkalitartat vorhanden ist.

3. Verfahren nach Anspruch 1 oder 2,
**dadurch gekennzeichnet,** daß
man einen Puffer verwendet, der zusätzlich modifiziertes Thermo-RSA, vorzugsweise in einer Menge von 0, 2 bis 0, 5 g/l, enthält.

4. Verfahren nach einem der vorhergehenden Ansprüche, bei welchem die Schritte
a) Kopplung eines für (1) PSA oder (2) ACT spezifischen Antikörpers an eine feste Phase,
b) Zugabe einer zu untersuchenden Probe zu der festen Phase und
c) Nachweis des Vorhandenseins von PSA-ACT-Komplexen durch Zugabe eines markierten Antikörpers, der im Fall a) (1) für ACT und im Fall a) (2) für PSA spezifisch ist und Detektion über die Markierung dieses Antikörpers,
durchgeführt werden,
**dadurch gekennzeichnet,** daß
zumindest in Schritt b) ein Puffer zugegen ist, mit Hilfe dessen die Testflüssigkeit auf einen Gehalt an 0,5 bis 2,5 mol/l eines Alkalichlorids eingestellt wird.

5. Puffersystem für ein heterologes, immunologisches Nachweisverfahren zur Bestimmung von PSA-ACT-Komplexen in Körperflüssigkeiten, enthaltend neben anderen üblichen Puffersubstanzen 0,5 bis 2,5 mol/l, vorzugsweise 1,5 bis 2,0 mol/l Alkalichlorid.

6. Puffersystem nach Anspruch 5,
**dadurch gekennzeichnet,** daß
es zusätzlich 0,01 bis 0,05 mol/l, vorzugsweise 0,02 bis 0,03 mol/l Alkalitartrat enthält.

7. Puffersystem nach einem der Ansprüche 5 oder 6,
**dadurch gekennzeichnet,** daß
es zusätzlich modifiziertes Thermo-RSA enthält.

8. Verwendung eines Puffersystems nach einem der Ansprüche 5 bis 7 in einem heterologen, immunologischen Nachweisverfahren für PSA-ACT-Komplexe in einer zu untersuchenden Probe.

9. Testkit für den Nachweis von PSA-ACT-Komplexen im Serum von Patienten,
**dadurch gekennzeichnet,** daß es
a) einen für PSA-spezifischen Antikörper und
b) einen für ACT-spezifischen Antikörper enthält, wobei einer der Antikörper a) und b) an eine feste Phase bindbar oder gebunden ist, und der andere Antikörper a) oder b) eine Markierung aufweist, über die die Nachweisreaktion erfolgt, und als Puffer mindestens ein Puffersystem nach einem der Ansprüche 5 bis 7 vorhanden ist.
